# EUROPEAN PATENT APPLICATION

(11) **EP 2 332 584 A1**
(43) Date of publication of application: **15.06.2011**
(21) Application number: 09425486.9
(22) Date of filing: 26.11.2009
(51) Int. Cl.: A61L 9/12, A61L 2/18, A61L 2/00, B65B 55/02, B67C 7/00

(54) **Method for sterilising containers**

(71) Applicant: Gea Procomac S.p.A., 43038 Sala Baganza (PR) (IT)
(72) Inventor: Pagliarini, Paolo, 43038 Sala Baganza (Parma) (IT); Casappa, Luigi, 43038 Sala Baganza (Parma) (IT)
(74) Representative: Lissandrini, Marco

(57) **Abstract**

A method for sterilising containers, comprising the following steps: preparing the containers to be sterilised; sterilising the containers with a mixture of water and a sterilising substance, inside an aseptic environment; rinsing the sterilised containers with sterile water; collecting the rinse fluid at the end of said rinsing step, said rinse fluid being a mixture of sterile water and a residual sterilising substance flushed out from the containers; controlling the flow of sterile water during said rinsing step so as to clean principally an interior surface of the containers; and using the rinse fluid for further operative steps of sterilisation or rinsing, without any intermediate step of removing the residual sterilising substance from the rinse fluid, so that the latter is an immediately recyclable sterilising mixture

The invention may be used, in particular, in the packaging sector for "sensitive" food products, i.e. products that are particularly susceptible to bacterial contamination and oxidation.

## Description

The present invention relates to a method for sterilising containers of the type described in the preamble of claim 1.

The invention can be employed in the packaging sector for "sensitive" food products, i.e. products that are particularly susceptible to bacterial contamination and oxidation, such as isotonic beverages, fruit juices and nectars, soft drinks, tea, milk-based beverages, coffee-based beverages and the like. For such products it is fundamental to avoid, during all stages of packaging, any contact with contaminating substances or with contaminated containers.

In the controlled atmosphere bottling sector, systems operating with "aseptic technology" are known. The latter is characterised in that the operations are carried out in an environment in which the microbiological load is controlled so as to assure a longer shelf life, according to the type of product, supply chain management procedures being equal.

As is known, a method for bottling under aseptic conditions always includes a step of sterilising the containers, irrespective of the further operative steps and technical solutions that may be adopted.

Typically, in the realm of "wet" technologies, said sterilisation step is performed by delivering a mixture of water and peracetic acid in a suitable concentration. Following the sterilisation step, there is provided a step of rinsing the containers to eliminate the residues of peracetic acid, as they are harmful to consumers' health and deleterious for the organoleptic characteristics of the products.

At present, the step of rinsing the containers is carried out in rinsing machines and is performed by using sterile water.

According to a first known technique, there is provided a step of recovering the water used to rinse the containers (collected water). In particular, at the end of the rinsing step, the water used is first conveyed into collection tanks situate at the base of the rinsing machine and then sent to a system for removal of the chemical substances. In said removal system, the collected water is submitted to a step of purification/filtration, typically by means of activated carbon, to eliminate the residues of peracetic acid, followed if necessary by a step of passing it through a reverse osmosis membrane so as to obtain water with suitable chemical / physical characteristics for subsequent reuse. The water then undergoes a sterilisation step (heat, microfiltration, UV, etc.) which restores the conditions of sterility thereof, thus enabling it to be recycled.

The sterilisation method summarily described above presents a major drawback in that it requires a complicated step of purifying/filtering the collected water via a system for removal of the chemical substances. Disadvantageously, after being purified/filtered, the collected water must also undergo a sterilisation treatment (e.g. by heat) that will restore it to conditions of sterility such that it can be reused in a subsequent rinsing or sterilisation step. In the latter case, however, the collected water that has been purified/filtered and sterilised cannot be immediately used, but must rather be suitably mixed with a sterilising substance in order to form the mixture necessary to sterilise the containers. Disadvantageously, a method according to the first known technique entails significant operating costs, in particular for maintenance of the removal system, especially for the replacement of activated carbon filters and for osmosis modules, if present.

According to a second known technique, in cases where the consumption of sterile water for the rinsing step is particularly limited, it is envisaged that the water used for rinsing will not be recovered, but rather directly disposed of, it being non-cost effective to implement the complex recovery methods described in reference to the first known technique.

There also exists a third known technique, which provides for disposal of the first bottle rinsing water (which has the highest chemical concentration) and recovery of the subsequent phase, to be sent to a facility for the removal of chemical residues. Disadvantageously, a sterilisation method according to the second or third known technique entails in any case a significant waste of water, above all if the system in which it is implemented operates on a continuous basis for long periods of time.

In this context, the technical task at the basis of the present invention is to provide a method for sterilising containers which overcomes the limitations of the above-mentioned known art.

In particular, it is an object of the present invention to provide a method for sterilising containers which enables water consumption to be reduced.

Another object of the present invention is to propose a method for sterilising containers whose implementation allows reducing the bulky dimensions and complexity of the systems currently used for the sterilisation of containers.

A further object of the present invention is to provide a method for sterilising containers that is repeatable and reliable.

Another object of the present invention is to provide a method for sterilising containers that can be implemented in bottling plants operating with aseptic technology.

The defined technical task and the specified objects hereof are substantially achieved by a method for sterilising containers which comprises the technical characteristics described in one or more of the appended claims.

Further characteristics and advantages of the present invention will become more apparent from the following approximate, and hence non-restrictive, description of a preferred, but not exclusive, embodiment of a method for sterilising containers.

A method for sterilising containers according to the invention can be employed, in particular, in the aseptic bottling sector, especially in the filling of PET bottles with "sensitive" beverages, such as isotonic beverages, fruit juices and nectars, soft drinks, tea, milk-based beverages, coffee-based beverages and the like.

Further characteristics and advantages of the present invention will become more apparent from the approximate, and hence non-restrictive, description of a preferred, but not exclusive, embodiment of a method for sterilising containers as illustrated in the diagram of the sole appended figure.

According to the invention, a method 1 for sterilising containers comprises the following steps:
- preparing the containers to be sterilised;
- sterilising 2 the containers with a mixture of water and a sterilising substance;
- rinsing 3 the sterilised containers with sterile water inside an aseptic environment;
- collecting 4 the rinse fluid at the end of the rinsing step.

It should be noted that the rinse fluid is a mixture of sterile water and a residual sterilising substance flushed out from the containers.

In the preferred embodiment, the sterilising substance is a liquid, preferably peracetic acid. It should be observed, however, that other liquids could be used to sterilise the containers, e.g. hydrogen peroxide. Advantageously, the method comprises the following steps:
- controlling the flow of sterile water during the rinsing step so as to clean principally an interior surface of the containers;
- using the rinse fluid for further operative steps of sterilisation or rinsing, without any intermediate step of removing the residual sterilising substance from the rinse fluid, so that the latter is an immediately recyclable sterilising mixture.

Given that, during the rinsing step, the flow of sterile water is controlled in such a way as to clean principally an interior surface of the containers, the residual sterilising substance flushed out from the containers is mixed with said flow of sterile water in quantities such as to ensure that the rinse fluid will have a sufficient concentration for subsequent sterilisation and rinsing operations.

In this respect, it may be observed that the concentration of the rinse fluid ranges between about 100 and about 250 ppm of peracetic acid. In other words, the concentration of the sterilising substance in the rinse fluid is 250 millilitres per litre of fluid. Advantageously, the method according to the invention enables water consumption to be reduced, since the further operative steps of sterilisation or rinsing are carried out using the sterilising mixture created after the container rinsing step.

The method according to the invention is simpler to implement compared to the known methods, as the intermediate step of removing the sterilising substance from the rinse fluid has been eliminated. Advantageously, therefore, the operating and maintenance costs of a system which implements the method of the invention are greatly reduced, it not being necessary to have any system for removing the sterilising substance from the rinse fluid. In particular, with reference to the first known technique described, no system for removal of the chemical substances is necessary. In this respect, given that the removal system typically relies on an activated carbon filter, a method according to the invention simplifies the system maintenance operations by avoiding the need to replace and periodically check the filter, while at the same time eliminating both the microbiological risks tied to the dimensions and geometries of the activated carbon filter and the inevitable consumption of water necessary for periodic counter-flushing of the activated carbon masses.

In cases where the sterilising mixture created after the container rinsing step is sufficiently concentrated for applications requiring limited microbial removal, the method further comprises a step 5 of sterilising closure capsules for the containers by spraying with said rinse fluid (connector 5a). Alternatively, i.e. in cases where the aforesaid mixture is not sufficiently concentrated for applications which require significant microbial removal, it is envisaged to use the rinse fluid (connector 6a) for a subsequent step 6 of rinsing the capsules.

Preferably, the operative fluid used to rinse the capsules is in turn recovered (connector 6b) and sent to a station 7 for diluting the peracetic acid (or another sterilising substance), in order to be made available again for the container sterilisation step. It should be noted that the operative fluid derives from said rinse fluid, being nothing other than a mixture between said rinse fluid and a sterilising substance used to sterilise the capsules.

The method may further comprise a step of cleaning the fumes released at the end of the container sterilisation step by means of said container rinse fluid (connector 8a). In this manner, an advantageous further recovery of peroxides will be obtained after the subsequent reuse (connector 8b) of the fluid itself in the peracetic acid dilution station and at the same time this will eliminate the need for dedicated towers to clean the fumes before they are emitted into the atmosphere.

The method may further comprise a step 9 of rinsing a neck of the containers by means of said rinse fluid (connector 9a) collected at the end of the container rinsing step.

Preferably, in addition or as an alternative to the preceding steps, the method may further comprise a step of rinsing a base of a container processing machine (connectors 10a, 9b, 11a) by means of said rinse fluid. Typically, the base submitted to the rinsing step can be the one supporting a container filling carousel (block 10) and/or that of a container neck rinsing carousel (block 9) and/or that of a capping carousel (block 11). According to a variant embodiment, and for the sole purpose of assuring a safer treatment, the method may further comprise a step 12 of irradiating the rinse fluid with a radiation source, in order to achieve a reduction/activation of the sterilising substance contained therein. Preferably, the irradiation step is performed using an ultraviolet ray source. In this regard, if the sterilising substance used is peracetic acid, said rinse fluid will contain a significant amount of peroxides, which are particularly reactive in the presence of ultraviolet radiation.

The method may further comprise a step 13 of micro-filtering the sterile water before said container rinsing step.

Preferably, the microfiltration step is performed using filters capable of selecting particles or other contaminating agents having a characteristic dimension of less than one micron.

Advantageously, a method for sterilising containers according to the invention is repeatable and extremely reliable.

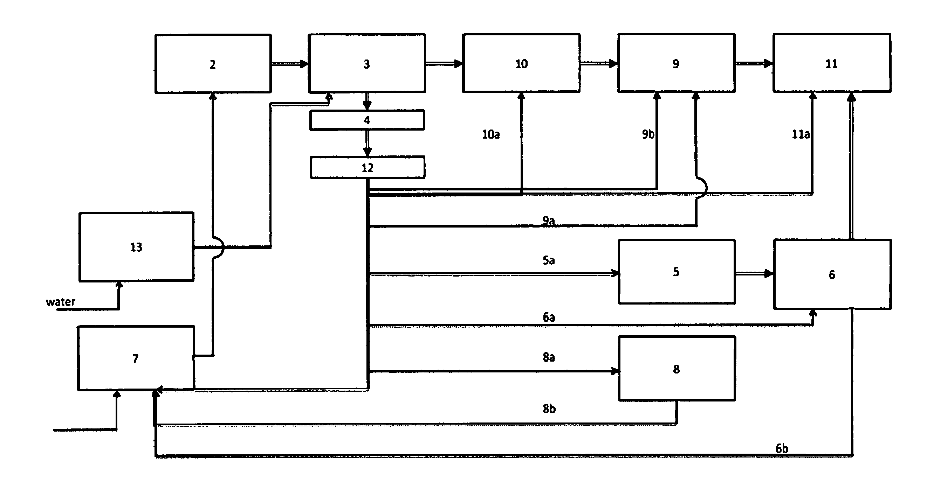

## Claims

1. Method (1) for sterilising containers, comprising the following steps:
- preparing the containers to be sterilised;
- sterilising (2) the containers with a mixture of water and a sterilising substance;
- rinsing (3) the sterilised containers with sterile water inside an aseptic environment;
- collecting (4) the rinse fluid at the end of said rinsing step, said rinse fluid being a mixture of sterile water and a residual sterilising substance flushed out from the containers,
**characterised in that** it comprises the following steps:
- controlling the flow of sterile water during said rinsing step so as to clean principally an interior surface of the containers;
- using the rinse fluid for further operative steps of sterilisation or rinsing (5, 6, 8, 9, 10, 11), without any intermediate step of removing the residual sterilising substance from the rinse fluid, so that the latter is an immediately recyclable sterilising mixture.

2. Method according to claim 1, further comprising a step of sterilising (5) closure capsules for the containers by spraying with said rinse fluid.

3. Method according to claim 1, further comprising a step of rinsing (6) closure capsules of containers by means of said rinse fluid.

4. Method according to any of the preceding claims, further comprising a step of rinsing (9) a neck of the containers by means of said rinse fluid.

5. Method according to any of the preceding claims, further comprising a step of rinsing (9b,10a,11a) a base of a container treatment machine by means of said rinse fluid.

6. Method according to any of the preceding claims, further comprising a step (12) of irradiating the rinse fluid with a radiation source, in order to achieve a reduction/activation of said sterilising substance.

7. Method according to claim 6, wherein said irradiation step (4) is performed using an ultraviolet ray source.

8. Method according to any of the preceding claims, further comprising a step (13) of micro-filtering the sterile water before said container rinsing step (3).

9. Method according to any of the preceding claims, further comprising a step (8) of cleaning fumes emitted at the end of said sterilisation step (2), prior to the emission thereof into the atmosphere, by means of said rinse fluid.

10. Method according to either of the claims 2 or 3, further comprising the following steps of:
- collecting the operative fluid used to treat said closure capsules (6), said operative fluid deriving from said rinse fluid;
- mixing (6b) said operative fluid with a predetermined quantity of sterilising substance, in order to obtain a sterilising mixture intended to be used in said container sterilisation step.

11. Method according to any of the preceding claims, wherein said sterilising substance comprises peracetic acid.
